# EUROPEAN PATENT APPLICATION

(11) **EP 3 340 091 A1**
(43) Date of publication of application: **27.06.2018**
(21) Application number: 16306816.6
(22) Date of filing: 26.12.2016
(51) Int. Cl.: G06F 19/00, B60K 28/06

(54) **METHOD AND MOBILE APPARATUS FOR MONITORING AND ANALYZING THE STATE OF HEALTH OF A PERSON**

(71) Applicant: Alcatel Lucent, 91620 Nozay (FR)
(72) Inventor: RAMACHANDRAN, Krishnamurthy, 600032 CHENNAI (IN)
(74) Representative: Shamsaei Far, Hassan

(57) **Abstract**

A method and a mobile apparatus for monitoring and analyzing a state of health of a person. A mobile communication apparatus is configured to obtain one or more samples of a person's breath using a breath analyzer installed a mobile communications apparatus. The breath analyzer analyzes the one or more samples of breath and generates data corresponding to a state of health of the person. The mobile communication apparatus transmits a signal to a processor of a vehicle using a wireless communication link, the signal containing processed information based on said generated data and indicative of the state of health of the person.

## Description

The present invention relates to devices for monitoring and analyzing the status of health of a person.

### ACKGROUND

This section introduces aspects that may help facilitate a better understanding of the present disclosure. Accordingly, the statements of this section are to be read in this light and are not to be understood as admissions about what is in the prior art or what is not in the prior art.

Unsafe driving, particularly drunk driving is often considered as a health hazard. The automotive industry has made great progress in improving the safety features of the vehicles they manufacture. In parallel great advances have also been achieved in designing vehicles with more powerful engines. In order to safely drive the increasingly powerful automobiles put in the market every year, the drivers may need to be physically fit. Indeed, any detrimental effect in the state of health of a driver can impair his or her driving capabilities which in turn may produce risks of accidents causing damages, personal injuries or loss of lives.

### SUMMARY

Some embodiments feature a mobile communications apparatus comprising a breath analyzer configured to obtain one or more samples of a person's breath and analyze said one or more samples to thereby generate data corresponding to a state of health of the person; wherein the mobile communication apparatus is configured to transmit a signal to a processor of a vehicle using a wireless communication link, said signal containing processed information based on said generated data and indicative of the state of health of the person.

According to some specific embodiments, the mobile communication apparatus is configured to receive a wireless alert signal initiated from said processer of the vehicle, said alert signal being indicative of an attempt to activate an ignition element of the vehicle.

According to some specific embodiments, the mobile communication apparatus is configured to process said generated data corresponding to a state of health of the person and to thereby determine whether said state of health of the person is within safe driving limits.

According to some specific embodiments, the mobile communication apparatus is configured to transmit said generated data to a remote server, and receiving said processed information from said remote server.

According to some specific embodiments, the mobile communication apparatus is configured to transmit said signal indicative that the state of health of the person to the processors of the vehicle, said signal containing instructions that the vehicle may be allowed to be operated by the person if said state of health of the person is assessed to be within safe limits of driving.

According to some specific embodiments, the mobile communication apparatus is configured to transmit said signal indicative that the state of health of the person to the processors of the vehicle, said signal containing instructions that the vehicle may not be allowed to be operated by the person if said state of health of the person is assessed to be out of safe limits of driving.

According to some specific embodiments, the mobile communication apparatus is configured to transmit the generated data corresponding to a state of health of the person to a server external to said mobile communication apparatus and said vehicle.

According to some specific embodiments, the mobile communication apparatus is configured to transmit said signal containing processed information based on said generated data and indicative of the state of health of the person to a server external to said mobile communication apparatus and said vehicle.

According to some specific embodiments, the mobile communication apparatus is configured to perform a biometric check of the person.

According to some specific embodiments, the mobile communication apparatus is configured to provide one or more pieces of information related to the processed information to the person.

According to some specific embodiments, said one or more pieces of information are one or a combination of: result of the breath analysis; whether or not such result is within the allowed or legal limits for driving; external entities notified with said one or more pieces of information; whether or not the vehicle is inhibited from operating.

According to some specific embodiments, the mobile device is configured to take one or more samples of the person's breath and analyze said one or more samples at regular intervals of time.

According to some specific embodiments, the mobile communication apparatus is configured to take one or more samples of the person's breath and analyze said one or more samples upon detecting a position of the vehicle using a localization application installed in the mobile communication apparatus.

Some embodiments feature a method of monitoring and analyzing a state of health of a person, comprising:
- obtaining one or more samples of a person's breath using a mobile communications apparatus comprising a breath analyzer;
- analyzing said one or more samples of breath and generating data to be processed corresponding to a state of health of the person;
- transmitting a signal to a processor of a vehicle using a wireless communication link, said signal containing processed information using said generated data and indicative of the state of health of the person.

According to some specific embodiments upon receiving an alert signal from the vehicle, the mobile communication apparatus initiates said breath analysis process by activating said breath analyzer module in order to obtain one or more samples of the person's breath.

According to some specific embodiments, the mobile communication apparatus transmits a signal to a processor of a vehicle using said wireless communication link, said signal containing processed information using said generated data and indicative of the state of health of the person.

According to some specific embodiments, the mobile communication apparatus transmits said signal indicative that the state of health of the person containing instructions that the vehicle may be allowed to be operated by the person if said state of health of the person is assessed to be within safe limits of driving.

According to some specific embodiments, the mobile communication apparatus transmits said signal indicative that the state of health of the person containing instructions that the vehicle may not be allowed to be operated by the person if said state of health of the person is assessed to be out of safe limits of driving.

These and further features and advantages of the present invention are described in more detail, for the purpose of illustration and not limitation, in the following description as well as in the claims with the aid of the accompanying drawings.

### BRIEF DESCRIPTION OF THE DRAWINGS

Figure 1 is a schematic representation of an example of an arrangement in which the apparatus of the present disclosure may be employed according to some embodiments.
Figure 2 is a flow chart representation of the method of monitoring and analyzing the state of health of a person according to some embodiments.

### DETAILED DESCRIPTION

As mentioned above, in view of the fabrication of increasingly more powerful vehicles, the need to ensure that the drivers are in an adequate state of health for driving such vehicles may become a priority. One common situation in which the state of health of a driver may be inadequate for driving is when the driver is driving under the influence of alcohol. Driving under the influence of alcohol, also known as drunk driving, may be the cause of many accidents. However, although drunk driving is one of the major causes of accidents, it is not the only cause for automobile accidents. In occasions, even if the driver is not under the influence of alcohol, the state of health of the driver may worsen due certain respiratory diseases such as lung cancer, tuberculosis or the like. A driver with such diseases may, at certain times during driving a vehicle, suffer from a sudden attack of coughing or respiratory shortness that would hinder his or her capability to drive and thereby cause accidents.

Today, many countries have established laws to control and penalize drunk driving or other careless or reckless driving behaviors caused as a result of driving under the influence of alcohol. However, such laws are often not sufficient to avoid that an individual who is not fit for driving operates a vehicle. Furthermore, the inventor is not aware of any specific laws regulating the way persons with long diseases may drive vehicles to avoid risk of damages and accidents.

Breath analyzers are known devices which are used for analyzing the quality of the air exhaled from a human's lung. Such devices are configured to analyze a sample from a person's breath to detect any possible substances therein which would be indicative of an anomaly in the person's state of health.

The term "state of health" as used herein is to be understood in a broad sense such that it not only relates to the state of the person in consideration of any illness, disease or otherwise unhealthy situation of the person, but also in consideration as to whether or not the person is under the influence of alcohol or drugs, which implies a that the person may have a state of health which is not proper for driving.

The present discourse addresses the problem of detecting and analyzing the state of health of a person using a mobile device. In this regard the present disclosure proposes the use of a built-in breath analyses function in a mobile device.

In the following description of embodiments, reference is made to the use of a smartphone. However, the disclosure is not so limited and any other mobile device such as for example laptop computers or tablets may also be appropriate for the implementation of the solution as proposed herein.

As it is well-known, the use of smartphones has become increasingly expanded and large populations in many countries already possess at least one smartphone for personal or business use, or both.

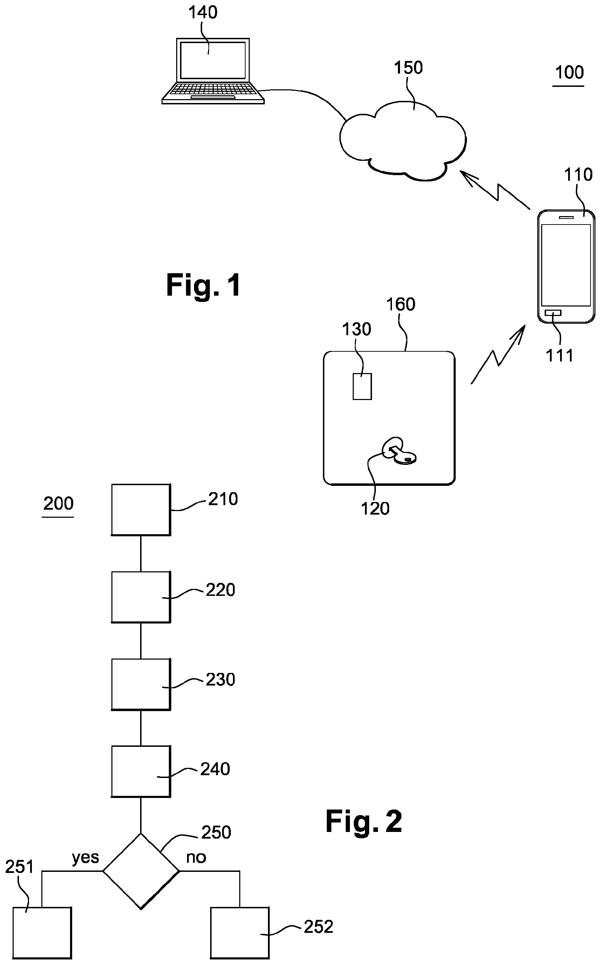

Figure 1 shows an arrangement 100 in which the proposed solution may be implemented. An exemplary possible scenario is disused below.

An individual (not shown) wishing to drive a vehicle 160 and carrying a smartphone 110 activates the ignition element 120 of the vehicle 160. In many cases, activating the ignition element may be done by inserting a key into an ignition slot located typically at some point near the steering wheel of the vehicle. In other occasions such ignition may be activated using "keyless" methods for example by pressing a start button or using a wireless connection between the key and the ignition element of the vehicle. Thus activating the ignition element is to be understood to cover all known means of performing such activation.

The vehicle 160 may be equipped with a processor 130 which controls the electronic operations of the vehicles. The processor 130 maybe of any known type already used in vehicles and may be further configured to carry out the operations required as disclosed herein.

Once the activation of ignition is performed, the processor receives a signal indicative of such activation and accordingly transmits a wireless alert signal to the smartphone. Such wireless connection may be achieved by establishing any known wireless communication technique such as for example Bluetooth. Currently, many vehicles are already equipped with Bluetooth applications capable of establishing communication, often automatically upon activating the ignition, with smartphones or other devices within the vehicle. The processor may therefore command the Bluetooth facility within the vehicle 160 to establish such wireless communication link, if such link is not already automatically established, and transmit such alert signal.

According to the present disclosure, the smartphone 110 comprises a breath analyzer module 111. The breath analyzer module 110 may use any known technique for detecting and analyzing a person's breath. The breath analyzer module 111 maybe based on the use of hardware integrated in the smartphone or it may be based on a software application embedded in the smartphone

Upon receiving such wireless alert signal, the smartphone 110 activates the breath analyzer module 111 in order to obtain one or more samples of the individual's breath.

In some embodiments, the breath samples may be taken by requesting the individual to provide samples of her/his breath (e.g. by generating visual and/or audio request). In this case, upon receiving such request, the individual may voluntarily provide one or more samples of her/his breath to the smartphone.

In some embodiments, in order to take the breath samples, the operation of the vehicle 160 may first be inhibited until such sample taking is performed. In other words, the vehicle 160 would not be able to operate (e.g. move) unless the state of health of the individual has been confirmed as being apt for driving. This option would not rely on the individual's willingness to cooperate in providing breath samples, but it would in practice oblige the individual to provide samples of breath in order to be able to eventually operate the vehicle.

In some preferred embodiments, although the ignition system is activated (as described above), thus the electronics of the vehicle 160 are operative, the engine may be inhibited from starting (thus the vehicle cannot move) until confirmation regarding the state of health of the individual is provided.

Once a sample of the breath of the individual is taken, the sample is analyzed by the breath analyzer 110 to detect the state of health of the individual (e.g. measuring the level of alcohol or detecting elements indicative of any disease in the individual's body). This analysis generates a corresponding data which can be transmitted by the smartphone 110 through wireless communication. In order to do this, the smartphone 110 may establish a wireless communication with a remote server 140 using a wireless communication network 150, based on known techniques such as for example the so-called 4G or 5G mobile communication techniques.

The remote server 140 may belong to any authority or organization which may have certain interest or control over the individual's driving activity. For example, one such remote serve 140 may belong to law enforcement authorities, such as police stations. Alternatively, or in addition, a remote server 140 may belong to an organization where the individual is employed or is in working relationship. For example, in such latter case, such organization may be a taxi aggregator company, e.g. Uber, for which the individual works as a taxi driver. Other non-limiting examples may include public transport companies, merchandise shipping companies (e.g. employing truck drivers), school bus companies, and the like.

In some embodiments, the smartphone 110 itself may be configured to determine whether the data corresponding to the state of health of the individual is within the safe driving limits to thereby allow the individual to operate the vehicle. In such case, the smartphone 110 may be provided with data pre-stored therein, such as look up tables or the like, and may be configured to compare the data obtained on the state of health of the individual with the pre-stored data and determine whether or not the individual's state of health is within the safe limits of driving. In either case, the smartphone 110 may be configured to send a signal to the vehicle's processor 130 using said wireless communication link which, after appropriate processing, is provided to the processor 130 in the form of usable data.

In some embodiments, the smartphone 110 itself may not be configured perform assessment regarding the state of health of the individual. In such case, the smartphone 110 may transmit data indicative of the state of health of the individual to a further processing unit (not shown). Such processing unit may be the above-mentioned remote serve 140 (belonging to a specific entity, e.g. law enforcement authorities or employment related organizations), or it may be an independent server, optionally being accessible by some or all interested entities. Such server therefore may be configured to process such data and provide further instructions to the smartphone 110.

Irrespective of whether or not the assessment of the state of health of the individual has been performed by the smartphone 110 itself or within an external server, the smartphone 110 transmits a signal containing appropriate instructions to the processor 130. Such signal may contain information indicative of the state of health of the individual based on processing the data (by the smartphone or an external server) generated by the smartphone upon performing the analysis of the breath of the individual. For example, the corresponding actions undertaken by the smartphone 110 may be the following:
- If the assessed state of health is found to be within the safe limits of driving, the smartphone may transmit a signal to the processor 130 indicating that the vehicle 160 may be allowed to operate by the individual, e.g. the engine may be enabled to start.
- If the assessed state of health is found to be out of the safe limits of driving, the smartphone may transmit a signal to the processor 130 indicating that the vehicle 160 may not be allowed to operate by the individual, e.g. starting the engine may remain inhibited.

In various embodiments, the data regarding the state of health of the individual may be stored in the remote server, in the smartphone or in a separate storage location such as a data repository.

In some embodiments, the data regarding the state of health of the individual may be shared between servers belonging to various entities, such as for example employment organizations as well as law enforcement authorities.

The proposed solution has many advantages as it may allow effective control of the driving conditions of drivers and therefore avoid or reduce the risk of accidents.

For example, as mentioned above, the processor 130 may be configured to enable or disable the operation of the vehicle 160 depending on the type of instructions received from the smartphone 110. Various scenarios may be considered:
- If the engine is not operating, the processer may generate appropriate commands to maintain the engine inoperative, for example during a certain period of time or until a further breath sample taken from the individual shows that his or her state of health has changed from previous "unsafe" condition to a safe condition whereby he or she can be allowed to drive.
- If the engine is operating, but the vehicle is not moving (e.g. standing in park position) the processer may generate appropriate commands to stop the operation of the engine and further follow-up may be performed, e.g. similar to the previous scenario.
- If the engine is operating and vehicle is moving, the processer may generate appropriate commands to alert the driver to stop as soon as possible or take any other appropriate action toward ensuring safety.

A further possibility may be prompting a second driver, if available, to replace the inhibited driver and a corresponding assessment may be performed on the state of health of the second driver together with corrective actions in a similar manner as described above.

In addition to the above safety or corrective measures, the entities having interest in the state of health of the individual may take additional actions. For example, a taxi aggregator agency may take appropriate corrective actions on the driver - such as issuing warnings or even delisting the driver in case a certain threshold of alcohol warnings is breached. Similarly, a law enforcement agency can decide to temporarily suspend or even permanently cancel the driving license of the individual based on the collected data and the severity of the situation.

In case of public transport drivers, a record of previous measurements of the state health of the drivers may be stored and made available to any party wishing to obtain knowledge therefrom. In case of taxi services, with every booking of a taxi, such information may be made available to the customer. These measures can serve as a deterrent for the driver to operate a vehicle under the influence of alcohol or under adverse health conditions. Such measures may also provide the customer with details that are relevant for taking an informed decision as to whether or not to accept the offered taxi service.

In some embodiments, a biometric check of the individual being tested may be performed associated with the breath analyzing process to ensure that the assessment is indeed done with respect to the identified driver and not on a different person falsely identified as the driver. For example, the biometric check is associated with the breath analysis process if it is performed during the analysis, although it may be performed prior, during or after taking samples of breath during a specific breath analysis session. This will help avoiding possible frauds during the assessment process. Such biometric checking may include, for example, face recognition, finger print recognition, voice recognition or other known authentication/recognition techniques.

The smartphone 110 may be configured to provide all or selected pieces of information related to the assessment process to the individual being assessed. For example, the smartphone may display information on the screen and/or generate sound messages indicating the result of the breath analysis, whether or not such result is within the allowed or legal limits for driving, which entities, if any, have been notified regarding the results of such assessment and whether or not the vehicle is inhibited from operating.

Figure 2 illustrates a flow chart representing a method 200 for monitoring and analyzing the health condition of a person according to some embodiments.

The method includes activating the ignition element of a vehicle, 210. Such activation of the ignition element may be performed in any known manner.

Upon activation of ignition, a wireless connection is established between the vehicle and the smartphone receive an alert signal from the vehicle, 220. Such wireless connection may be achieved by establishing any known wireless communication technique such as for example Bluetooth. The wireless communication may be established using a processor in the vehicle configured to command a Bluetooth facility within the vehicle to establish such wireless communication and transmit such alert signal.

Upon receiving such alert signal, the smartphone initiates a breath analysis process, 230. Such breath analysis may be performed by activating a breath analyzer module in order to obtain one or more samples of the individual's breath.

Once a sample of the breath of the individual is taken, the sample is analyzed by the breath analyzer to detect the state of health of the individual (e.g. measuring the level of alcohol or detecting elements indicative of any disease in the individual's body), and data corresponding to the detected state of health of the individual is generated 240.

Next the data corresponding to the state of health of the individual is assessed, 250, so as to determine whether such data is within such limits which would correspond to safe driving limits to thereby allow the individual to operate the vehicle.

If the assessed state of health is found to be within the safe limits of driving, 251, the smartphone transmits a signal to the processor of the vehicle indicating that the vehicle may be allowed to operate by the individual, e.g. the engine may be enabled to start.

If the assessed state of health is found to be out of the safe limits of driving, 252, the smartphone transmits a signal to the processor of the vehicle indicating that the vehicle may not be allowed to operate by the individual, e.g. starting the engine may remain inhibited.

In some embodiments, the breath samples may be taken by requesting the individual to provide samples of her/his breath.

In some embodiments, in order to take the breath samples, the operation of the vehicle may first be inhibited until such sample taking is performed.

In some preferred embodiments, although the ignition system is activated (as described above), thus the electronics of the vehicle are operative, the engine may be inhibited from starting (thus the vehicle cannot move) until confirmation regarding the state of health of the individual is provided.

In some embodiments, the determination as to whether the data corresponding to the state of health of the individual is within the safe driving limits is performed by the smartphone itself.

In some embodiments, the determination as to whether the data corresponding to the state of health of the individual is within the safe driving limits is performed by a further processing unit such that said data indicative of the state of health of the individual is transmitted to such further processing unit.

In some embodiments, such further processing unit may be a remote serve belonging to a specific entity, e.g. law enforcement authorities or employment related organizations.

In some embodiments, such further processing unit may be an independent server, optionally being accessible by some or all interested entities. Such independent server therefore may be configured to process such data and provide further instructions to the smartphone.

In various embodiments, the data regarding the state of health of the individual may be stored in the remote server, in the smartphone or in a separate storage location such as a data repository.

In some embodiments, the data regarding the state of health of the individual may be shared between servers belonging to various entities, such as for example employment organizations as well as law enforcement authorities.

In some embodiments, a biometric check of the individual being tested may be performed together with the breath analyzing process to ensure that the assessment is indeed done with respect to the identified driver and not on a different person falsely identified as the driver. This will help avoiding possible frauds during the assessment process. Such biometric checking may include, for example, face recognition, finger print recognition, voice recognition or other known authentication/recognition techniques.

In some embodiments, all or selected pieces of information related to the assessment process are provided by the smartphone to the individual being assessed.

In some embodiments, the smartphone may be configured to initiate, without receiving an alert signal from the processor of the vehicle, a process of taking of one or more samples of the person's breath and analyze said one or more samples at regular intervals of time, for example to ensure that the driver has not taken alcohol during driving or while the engine is running, or to ensure that the state of health of the driver is maintained at a satisfactory level even after a relatively prolonged driving time.

In some embodiments, the smartphone may be configured to initiate, without receiving an alert signal from the processor of the vehicle, a process of taking of one or more samples of the person's breath and analyze said one or more samples based on detecting the position of the vehicle. This may be the case where the vehicle is in a relatively close vicinity of places where alcohol is served such as bars and restaurants. The proximity of the vehicle to these locations may be detected using available localization applications such as the Global Positioning System (GPS) which may be already installed on the smartphone. To this end, the smartphone may be configured to initiate a process of taking of one or more samples of the person's breath and analyze said one or more samples when the vehicle moves close to a predetermined location, e.g. a few hundred meters from a bar. This will serve the purpose of ensuring that the driver has not taken alcohol by getting close to such locations to drink while the engine is running.

Embodiments of the present disclosure may include blocks which can be hardware devices, software modules or combination of hardware devices and software modules

The various embodiments of the present invention may be combined as long as such combination is compatible and/or complimentary.

The method is implemented in a preferred embodiment through or together with a software process or software module including one or more software programs. Further it is to be noted that the list of structures corresponding to the claimed means is not exhaustive and that one skilled in the art understands that equivalent structures can be substituted for the recited structure without departing from the scope of the invention.

It is also to be noted that the order of the steps of the method of the invention as described and recited in the corresponding claims is not limited to the order as presented and described and may vary without departing from the scope of the invention.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## Claims

1. A mobile communications apparatus comprising a breath analyzer configured to obtain one or more samples of a person's breath and analyze said one or more samples to thereby generate data corresponding to a state of health of the person; wherein the mobile communication apparatus is configured to transmit a signal to a processor of a vehicle using a wireless communication link, said signal containing processed information based on said generated data and indicative of the state of health of the person.

2. The mobile communication apparatus of claim 1, configured to receive a wireless alert signal initiated from said processer of the vehicle, said alert signal being indicative of an attempt to activate an ignition element of the vehicle.

3. The mobile communication apparatus of claim 1 or claim 2, configured to process said generated data corresponding to a state of health of the person and to thereby determine whether said state of health of the person is within safe driving limits.

4. The mobile communication apparatus of claim 1 or claim 2, configured to transmit said generated data to a remote server, and receive said processed information from said remote server.

5. The mobile communication apparatus of any one of the preceding claims, configured to transmit said signal indicative that the state of health of the person to the processors of the vehicle, said signal containing instructions that the vehicle may be allowed to be operated by the person if said state of health of the person is assessed to be within safe limits of driving.

6. The mobile communication apparatus of any one of the preceding claims, configured to transmit said signal indicative that the state of health of the person to the processors of the vehicle, said signal containing instructions that the vehicle may not be allowed to be operated by the person if said state of health of the person is assessed to be out of safe limits of driving.

7. The mobile communication apparatus of any one of the preceding claims, configured to transmit the generated data corresponding to a state of health of the person to a server external to said mobile communication apparatus and said vehicle.

8. The mobile communication apparatus of any one of the preceding claims, configured to transmit said signal containing processed information based on said generated data and indicative of the state of health of the person to a server external to said mobile communication apparatus and said vehicle.

9. The mobile communication apparatus of any one of the preceding claims, configured to perform a biometric check of the person.

10. The mobile communication apparatus of any one of the preceding claims, configured to provide one or more pieces of information related to the processed information to the person.

11. The mobile communication apparatus of claim 10, wherein said one or more pieces of information are one or a combination of: result of the breath analysis; whether or not such result is within the allowed or legal limits for driving; external entities notified with said one or more pieces of information; whether or not the vehicle is inhibited from operating.

12. The mobile communication apparatus of claim 1, wherein the mobile device is configured to take one or more samples of the person's breath and analyze said one or more samples at regular intervals of time.

13. The mobile communication apparatus of claim 1, wherein the mobile communication apparatus is configured to take one or more samples of the person's breath and analyze said one or more samples upon detecting a position of the vehicle using a localization application installed in the mobile communication apparatus.

14. A method of monitoring and analyzing a state of health of a person, comprising:
- obtaining one or more samples of a person's breath using a mobile communications apparatus comprising a breath analyzer;
- analyzing said one or more samples of breath and generating data corresponding to a state of health of the person;
- transmitting a signal to a processor of a vehicle using a wireless communication link, said signal containing processed information based on said generated data and indicative of the state of health of the person.

15. The method of claim 12, wherein upon receiving an alert signal from the vehicle, the mobile communication apparatus initiates said breath analysis process by activating said breath analyzer module in order to obtain one or more samples of the person's breath.

16. The method of claim 12 or claim 13, wherein the mobile communication apparatus transmits a signal to a processor of a vehicle using said wireless communication link, said signal containing processed information using said generated data and indicative of the state of health of the person.

17. The method of any of the preceding claims 12-14, wherein the mobile communication apparatus transmits said signal indicative that the state of health of the person containing instructions that the vehicle may be allowed to be operated by the person if said state of health of the person is assessed to be within safe limits of driving.

18. The method of any of the preceding claims 12-14, wherein the mobile communication apparatus transmits said signal indicative that the state of health of the person containing instructions that the vehicle may not be allowed to be operated by the person if said state of health of the person is assessed to be out of safe limits of driving.
